(19) Europäisches Patentamt
European Patent Office
Office européen des brevets

(11) **EP 4 432 298 A1**

(12) **EUROPEAN PATENT APPLICATION**

(43) Date of publication:
**18.09.2024 Bulletin 2024/38**

(21) Application number: **24158485.3**

(22) Date of filing: **19.02.2024**

(51) International Patent Classification (IPC):
**G16H 40/60** (2018.01)   **G05B 21/00** (2006.01)
**G05B 23/00** (2006.01)

(52) Cooperative Patent Classification (CPC):
**G16H 40/60;** G05B 23/0283; G06Q 10/20

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **17.03.2023 IN 202311018137**

(71) Applicant: Honeywell International Inc.
**Charlotte, NC 28202 (US)**

(72) Inventors:
• **SREERAMU, Sudheer Beligere**
**Charlotte, 28202 (US)**
• **SAINI, Hitesh**
**Charlotte, 28202 (US)**
• **NARASIMHAN, Kumaran S**
**Charlotte, 28202 (US)**

(74) Representative: **Haseltine Lake Kempner LLP
Cheapside House
138 Cheapside
London EC2V 6BJ (GB)**

(54) **METHODS FOR DETECTING REUSE OF SENSING COMPONENT**

(57)     A method for detecting reuse of a sensing component by a controller component is provide. The method includes: reading device information of the sensing component; determining if the sensing component is reused based on the device information; in an instance in which the sensing component is reused, generating a reuse alert; and in an instance in which the sensing component is not reused, determining a life expectancy of the sensing component.

FIG. 1

EP 4 432 298 A1

**Description**

**FIELD OF THE INVENTION**

Exemplary embodiments of the present disclosure relate generally to methods for detecting reuse of a sensing component, and more particularly, in some examples, to methods for detecting a life expectancy of a sensing component.

**BACKGROUND**

Medically disposable sensors may include disposable sensing components and non-disposable controller components. In general, the non-disposable controller components can still be reused and the disposable sensing components may be replaced after each single use in order to maintain sterilization in a medical environment. However, the disposable sensing components may be reused intentionally or by mistake. Through applied effort, ingenuity, and innovation, many of these identified problems have been solved by developing solutions that are included in embodiments of the present disclosure, many examples of which are described in detail herein.

**BRIEF SUMMARY**

Various embodiments described herein relate to methods, apparatuses, and systems for detecting reuse and life expectancy of a sensing component such as, for example, a flow sensor component.
In accordance with various examples of the present disclosure, a method for detecting reuse of a sensing component by a controller component is provide. The method includes: reading device information of the sensing component; determining if the sensing component is reused based on the device information; in an instance in which the sensing component is reused, generating a reuse alert; and in an instance in which the sensing component is not reused, determining a life expectancy of the sensing component.
In some embodiments, determining if the sensing component is reused based on the device information includes: determining if the sensing component is connected to a server before; in an instance in which the sensing component has not been connected to the server before: uploading the device information to the server; querying a database of the server with the device information; determining if the device information exists in the database of the serve; and in an instance in which the device information exists in the database of the server, determining that the sensing component is reused.
In some embodiments, determining the life expectancy of the sensing component includes: uploading the device information to a server; collecting data from the sensing component for a predetermined period; determining a used life of the sensing component by analyzing the collected data with a binning method; and determining the life expectancy of the sensing component based on the device information and the used life.
In some embodiments, the device information includes an identification of the sensing component and parameters of the sensing component.
In some embodiments, the parameters of the sensing component includes an ambient temperature, an operating flow, and a transient flow of the sensing component.
In some embodiments, analyzing the collected data with the binning method composes: determining bin sizes and class intervals based on the collected data; determining peaks and valleys of the collected data; identifying corresponding bins of the peaks and the valleys of the collected data; counting a corresponding frequency of each of the identified corresponding bins; and calculating the used life of the sensing component 101 based on the identified corresponding bins and the corresponding frequency of each of the identified corresponding bins.
In some embodiments, the sensing component is a flow sensing component configured to measure a flow rate of a flowing media.
In some embodiments, the method further comprising connecting the sensing component with the controller component, where the sensing component is attachable to the controller component and the sensing component is configured to be in an electrical communication with the controller component when attached.
In some embodiments, the controller component includes a signal conditioner.
In some embodiments, the signal conditioner is an amplifier or an analog-to-digital converter (ADC).
In some embodiments, The method further includes: storing the life expectancy of the sensing component.
In some embodiments, the method further includes: updating the life expectancy of the sensing component on a server.
In some embodiments, the controller component is further configured to send the reuse alert in rea-time.
In accordance with various examples of the present disclosure, a sensor for detecting reuse of a sensing component is provided. The sensor includes: a sensing component, and a controller component, where the sensing component is attachable to the controller component, and the controller component is configured to: read device information of the sensing component; determine if the sensing component is reused based on the device information; in an instance in which the sensing component is reused, generate a reuse alert; and in an instance in which the sensing component is not reused, determine a life expectancy of the sensing component.

**BRIEF DESCRIPTION OF THE DRAWINGS**

The description of the illustrative embodiments may be read in conjunction with the accompanying figures. It will be appreciated that, for simplicity and clarity of illustration, elements illustrated in the figures have not necessarily been drawn to scale, unless described otherwise. For example, the dimensions of some of the elements may be exaggerated relative to other elements, unless described otherwise. Embodiments incorporating teachings of the present disclosure are shown and described with respect to the figures presented herein, in which:

FIG. 1 illustrates a schematic diagram depicting an example system for detecting reuse of a sensing component, in accordance with various embodiments of the present disclosure;
FIG. 2 illustrates a schematic diagram depicting an example remote computing server, in accordance with various embodiments of the present disclosure;
FIG. 3 illustrates a schematic diagram depicting an example controller component, in accordance with various embodiments of the present disclosure;
FIG. 4 illustrates a flowchart diagram illustrating an example method for detecting reuse of a sensing component, in accordance with various embodiments of the present disclosure is provided;
FIG. 5 illustrates a flowchart diagram illustrating an example method for determining if a sensing component is reused, in accordance with various embodiments of the present disclosure is provided;
FIG. 6 illustrates a flowchart diagram illustrating an example method for determining a life expectancy of the sensing component, in accordance with various embodiments of the present disclosure is provided;
FIG. 7 illustrates a flowchart diagram illustrating an example method for determining a used life of a sensing component, in accordance with various embodiments of the present disclosure is provided; and
FIG. 8 illustrates a diagram illustrating an example method for determining a used life of a sensing component, in accordance with various embodiments of the present disclosure is provided.

**DETAILED DESCRIPTION OF THE INVENTION**

Some embodiments of the present disclosure will now be described more fully hereinafter with reference to the accompanying drawings, in which some, but not all embodiments of the disclosure are shown. Indeed, these disclosures may be embodied in many different forms and should not be construed as limited to the embodiments set forth herein; rather, these embodiments are provided so that this disclosure will satisfy applicable legal requirements. Like numbers refer to like elements throughout.

The components illustrated in the figures represent components that may or may not be present in various embodiments of the present disclosure described herein such that embodiments may include fewer or more components than those shown in the figures while not departing from the scope of the present disclosure. Some components may be omitted from one or more figures or shown in a dashed line for visibility of the underlying components.

The phrases "in an example embodiment," "some embodiments," "various embodiments," and the like generally mean that the particular feature, structure, or characteristic following the phrase may be included in at least one embodiment of the present disclosure, and may be included in more than one embodiment of the present disclosure (importantly, such phrases do not necessarily refer to the same embodiment).

The word "example" or "exemplary" is used herein to mean "serving as an example, instance, or illustration." Any implementation described herein as "exemplary" is not necessarily to be construed as preferred or advantageous over other implementations.

If the specification states a component or feature "may," "can," "could," "should," "would," "preferably," "possibly," "typically," "optionally," "for example," "often," or "might" (or other such language) be included or have a characteristic, that a specific component or feature is not required to be included or to have the characteristic. Such components or features may be optionally included in some embodiments, or may be excluded.

The term "electronically coupled" or "in electronic communication with" in the present disclosure refers to two or more electrical elements (for example, but not limited to, an example processing circuitry, communication module, input/output module memory, humidity sensing component, cooling element, gas detection component) and/or electric circuit(s) being connected through wired means (for example but not limited to, conductive wires or traces) and/or wireless means (for example but not limited to, wireless network, electromagnetic field), such that data and/or information (for example, electronic indications, signals) may be transmitted to and/or received from the electrical elements and/or electric circuit(s) that are electronically coupled.

The term "flow sensor" may refer to an apparatus that may detect, measure, sense, and/or identify flow rate(s) (including, but not limited to, linear flow velocity, nonlinear flow velocity, mass flow rate, and/or volumetric flow rate) of a flowing media or medium. In the present disclosure, the term "flowing media" refers to a substance (such as, but not limited to, a liquid substance and/or a gaseous substance).

Various example embodiments address technical problems associated with methods to detect reuse, abuse, damage, or other wear and tear of a sensing component and a life expectancy of the sensing component. As understood by those of skill in the field to which the present disclosure pertains and in some examples, there are numerous example scenarios in which a user may need to detect reuse (abuse or some other damage) of the sensing component and the life expectancy of the sensing component.

In some examples, medical sensors have many compliance requirements coming from the food and drug administration (FDA) or other agency local to a particular jurisdiction and medical regulation calls for stringent environmental survival especially from sterilization processes before being deployed into and/or around a patient body. For example, traditional medically disposable sensors may include a sensing element and a controller component electrically coupled to the sensing element. During the sterilization processes, the controller component, which may include electronics, such as signal conditioning circuits, is more susceptible, tends to fail, have degraded performance, and/or the like. In some examples, the sensing element may be a disposable sensor, such that the sensing element is used only once, the patient will not be exposed to the contamination from a previous patient, and the sensing element does not need to be recalibrated due to previous exposure, in some examples. In alternative or additional examples, the controller component has no direct contact with the patient and may be reused.

However, the disposable sensing components, which are designed for a single use, in some examples, may be used multiple times intentionally or by mistake. For example, high errors may be introduced if the sensing components are reused and have already been exposed to a previous patient. In order to maintain sterilization in the medical environment, various example embodiments in the present disclosure described herein utilize various techniques to detect reuse, such as abuse, damage, wear, tear, and/or the like of the sensing component and a life expectancy of the sensing component. In some examples herein, the reuse of a sensing component may be classified as an abuse.

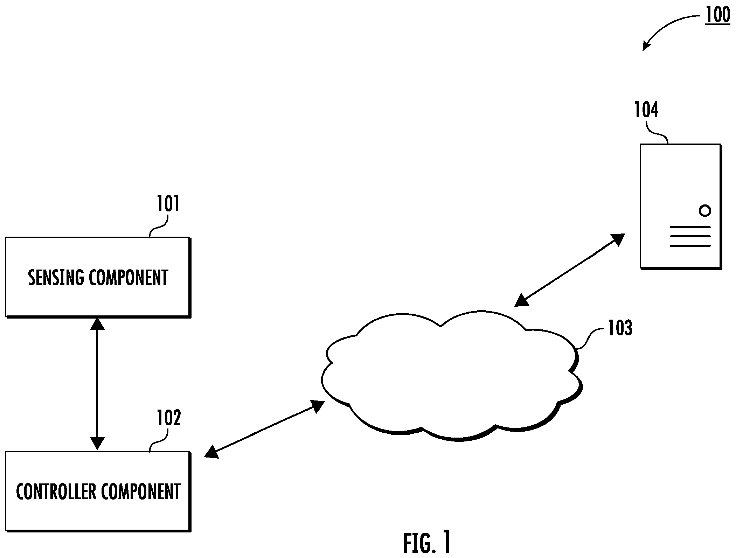

Referring now to FIG. 1, a schematic diagram depicting an example system for detecting reuse of a sensing component, in accordance with various embodiments of the present disclosure is provided. As depicted in FIG. 1, a system 100 for detecting reuse of a sensing component includes the sensing component 101, a controller component 102, one or more networks 103, and a remote computing server 104.

In some embodiments, each of the components of the example analytics and monitoring the system 100 may be in electronic communication with, for example, one another over the same or different wireless or wired networks 103 including, for example, a wired or wireless Personal Area Network (PAN), Local Area Network (LAN), Metropolitan Area Network (MAN), Wide Area Network (WAN), and/or the like. Additionally, while FIG. 1 illustrates certain system entities as separate, standalone entities, the various embodiments are not limited to this particular architecture.

For example, the controller component 102, and the remote computing server 104 in the remote computing platform may be in electronic communication with one another to exchange data and information. As described herein, the controller component 102 may receive data from the sensing component 101. In some embodiments, the controller component 102 may transmit data from the sensing component 101 to the remote computing server 104 in the remote computing platform for analysis. Data from the sensing component 101 may be distributed into individual monitoring channels corresponding to the sensing component 101.

In some embodiments, the remote computing server 104 in the remote computing platform may receive the data from the controller component 102 through the one or more networks 103 and may generate estimated characteristics data associated with the sensing component 101 based at least in part on the data. For example, the controller component 102 may transmit data to the remote computing server 104. Upon receiving the data, the remote computing server 104 may process the estimated characteristics data for rendering on a graphical user interface ("GUI"). In some embodiments, the remote computing server 104 may generate one or more data points on the GUI based on the data in accordance with various example methods described herein.

In various embodiments, the sensing component 101 may be a flow sensor component, a pressure sensing component, a temperature sensing component, or the like. For example, the sensing component 101 may a flow sensor component configured to detect, measure, and/or identify flow rate(s) (including, but not limited to, linear flow velocity, nonlinear flow velocity, mass flow rate, and/or volumetric flow rate) of a flowing media or medium. In some embodiments, the sensing component 101 may be electrically connected with and in communication with the controller component 102.

In some embodiments, the sensing component 101 may be a removable sensing component and may be disposed after a single use. For example, a flow sensing component, which may be dispensed into a human body and configured to measure a fluid volume, may be disposed after each single use in an equipment such as a hemodialysis machine, an infusion pump, and/or the like. In some examples, the sensing component 101 is attachable to the controller component 102. For example, the sensing component 101 may be configured to be in an electrical communication with the controller component when attached to the controller component 102. For example, the sensing component 101 may detect whether the sensing component 101 is reused. In additional examples, detecting reuse of the sensing component 101 may include detecting abuse, damage, wear, tear, and/or the like of the sensing component 101.

In some embodiments, the sensing component 101 may include device information of the sensing component 101. For example, the device information of the sensing component 101 may include a unique characteristic of an identification (ID). In some examples, the unique characteristic of an ID may be a serial number, a barcode, a quick response (QR) code, and/or the like.

In some embodiments, the controller component 102 may be configured to track and store the ID of the sensing component 101. In some embodiments, the controller component 102 may transmit the ID of the sensing component 101 to the remote computing server 104and the remote computing server 104may be configured to track and store the ID of the sensing component 101. For example, the remote computing server 104may track and store an identified, a serial number, a barcode, and/or a QR code for identification of the sensing component 101. In additional examples, the remote computing server 104may also track and store calibration parameters of the sensing component 101, such as an ambient temperature, an average operating flow, and a transient flow of the sensing component 101.

In various embodiments, the controller component 102 may determine if the sensing component 101 has previously been connected to a remote computing server 104. If the controller component 102 determines that the sensing component 101 has been previously connected to the remote computing server 104, the controller component 102 may be configured to upload the device information to the remote computing server 104. If the controller component 102 determines that the sensing component 101 has not been previously connected to the remote computing server 104, the controller component 102 may be configured to read a status of the sensing component 101 from the remote computing server 104.

In various embodiments, the controller component 102 may query a database of the remote computing server 104 with the device information. In various embodiments, the controller component 102 may determine if the device information exists in the database of the remote computing server 104. If the controller component 102 determines that the device information exists in the database of the remote computing server 104, the controller component 102 may determine that the sensing component is reused. If the controller component 102 determines that the device information does not exist in the database of the server, the controller component 102 may determine a life expectancy of the sensing component 101.

In some embodiments, the device information of the sensing component 101 may further include calibration parameters of the sensing component. For example, the calibration parameters of the sensing component may include an ambient temperature, an operating flow, and a transient flow of the sensing component. In some examples, the device information may be stored in the controller component 102.

In various embodiments, the controller component 102 may include signal conditioners. such as, but not limited to, an amplifier, an analog-to-digital converter (ADC), and/or the like. For example, the amplifier may be a weak-signal amplifier, which increases a signal amplitude of the sensing component 101. In some examples, a signal of the sensing component 101 may be an analog signal. In some embodiments, the ADC may convert the analog signal of the sensing component 101 to a digital signal to represent the signal of the sensing component 101.

In various embodiments, the controller component 102 may decode and convert the device information and the calibration parameters of the sensing component (an input signal of the controller component 102) to a flow measurement data (an output signal of the controller component 102). In some examples, the controller component 102 may be further configured to upload the flow measurement data to the remote computing server 104.

In various embodiments, the controller component 102 may be configured to generate a reuse alert in real-time in an instance in which the sensing component 101 is reused. In some examples, the reuse alert may be a visual indicator and/or an acoustic indicator. In some examples, the reuse alert may warn the users that the sensing component 101 is reused and the performance of the sensing component 101 may be affected.

In various embodiments, the controller component 102 may be configured to track a usage of the sensing component 101. For example, the controller component 102 may be configured to calculate a used life of the sensing component 101 by analyzing collected data from the sensing component 101.

For example, the sensing component 101 may be flow sensing component configured to measure a flow rate of a flowing media, in which the sensing component is exposed. In some examples, the controller component 102 may collect data of the flow rate continuously from the sensing component 101 for a predetermined period.

In some embodiments, the controller component 102 may determine the used life of the sensing component 101 by analyzing the collected data with a binning method. For example, the binning method is a way to group a number of more or less continuous values into a smaller number of bins. For example, the collected data may include a continuous flow rate in a range, and the arrange may be divided into a number of flow rate intervals, which is called bins. The collected data may be analyzed by counting a frequency of the flow rate in each of the bins.

In various embodiments, the used life of the sensing component 101 may be calculated based on the following equation (Eq. 1).

$$\text{used life} = n1/N1 + n2/N2 + n3/N3 + \dots \quad (\text{Eq.1})$$

For example, n1, n2, n3,... are the corresponding frequencies of each of the identified corresponding bins. N1, N2, N3,... are the corresponding coefficients of each of the identified corresponding bins. In some examples, each of the corresponding coefficients depends on a flow range of the corresponding bin and a range of the sensing component 101.

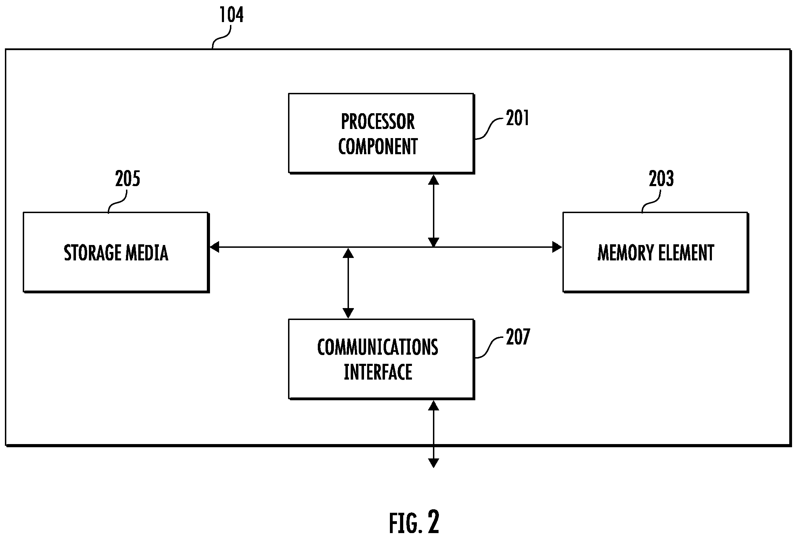

Referring now to FIG. 2, an example schematic representation of an example remote computing server 104 in an example remote computing platform in accordance with some example embodiments described herein. In some embodiments, the example remote computing platform may be a cloud computing platform, and the example remote computing server may be a cloud computing server.

As indicated, in some embodiments, the remote computing server 104 may include one or more network and/or communications interface 207 for communicating with various computing entities, such as by communicating data, content, information, and/or similar terms used herein that can be transmitted, received, operated on, processed, displayed, stored, and/or the like. For instance, the remote computing server 104 may communicate with controller component 102 through the one or more networks 103.

As shown in FIG. 2, in one embodiment, the remote computing server 104 may include or be in communication with one or more processor components (for example, processor component 201) (also referred to as processor components, processing circuitry, and/or similar terms used herein interchangeably) that communicate with other elements within the remote computing server 104 via a bus, for example, or network connection. As will be understood, the processor component 201 may be embodied in a number of different ways. For example, the processor component 201 may be embodied as one or more complex programmable logic devices (CPLDs), microprocessor components, multi-core processor components, co-processing entities, application-specific instruction-set processor components (ASIPs), and/or controllers. Further, the processor component 201 may be embodied as one or more other processing devices or circuitry. The term circuitry may refer to an entirely hardware embodiment or a combination of hardware and computer program products. Thus, the processor component 201 may be embodied as integrated circuits, application specific integrated circuits (ASICs), field programmable gate arrays (FPGAs), programmable logic arrays (PLAs), hardware accelerators, other circuitry, and/or the like. As will therefore be understood, the processor component 201 may be configured for a particular use or configured to execute instructions stored in volatile or non-volatile media or otherwise accessible to the processor component 201. As such, whether configured by hardware or computer program products, or by a combination thereof, the processor component 201 may be capable of performing steps or operations according to embodiments of the present disclosure when configured accordingly.

In one embodiment, the remote computing server 104 may further include or be in communication with volatile media (also referred to as volatile storage, memory, memory storage, memory circuitry and/or similar terms used herein interchangeably). In one embodiment, the volatile storage or memory may also include one or more memory element 203 as described above, such as RAM, DRAM, SRAM, FPM DRAM, EDO DRAM, SDRAM, DDR SDRAM, DDR2 SDRAM, DDR3 SDRAM, RDRAM, RIMM, DIMM, SIMM, VRAM, cache memory, register memory, and/or the like. As will be recognized, the volatile storage or memory element 203 may be used to store at least portions of the databases, database instances, database management system entities, data, applications, programs, program modules, scripts, source code, object code, byte code, compiled code, interpreted code, machine code, executable instructions, and/or the like being executed by, for example, the processor component 201 as shown in FIG. 2. Thus, the databases, database instances, database management system entities, data, applications, programs, program modules, scripts, source code, object code, byte code, compiled code, interpreted code, machine code, executable instructions, and/or the like may be used to control certain aspects of the operation of the remote computing server 104 with the assistance of the processor component 201 and operating system.

In one embodiment, the remote computing server 104 may further include or be in communication with non-volatile media (also referred to as non-volatile storage, memory, memory storage, memory circuitry and/or similar terms used herein interchangeably). In one embodiment, the non-volatile storage or memory may include one or more non-volatile storage or storage media 205 as described above, such as hard disks, ROM, PROM, EPROM, EEPROM, flash memory, MMCs, SD memory cards, Memory Sticks, CBRAM, PRAM, FeRAM, RRAM, SONOS, racetrack memory, and/or the like. As will be recognized, the non-volatile storage or storage media 205 may store databases, database instances, database management system entities, data, applications, programs, program modules, scripts, source code, object code, byte code, compiled code, interpreted code, machine code, executable instructions, and/or the like. The term database, database instance, database management system entity, and/or similar terms used herein interchangeably and in a general sense to refer to a structured or unstructured collection of information/data that is stored in a computer-readable storage medium.

Storage media 205 may also be embodied as a data storage device or devices, as a separate database server or servers, or as a combination of data storage devices and separate database servers. Further, in some embodiments, storage media 205 may be embodied as a distributed repository such that some of the stored information/data is stored centrally in a location within the system and other information/data is stored in one or more remote locations. Alternatively, in some embodiments, the distributed repository may be distributed over a plurality of remote storage locations only. An example of the embodiments contemplated herein would include a cloud data storage system maintained by a third-party provider and where some or all of the information/data required for the operation of the recovery prediction system may be stored.

As indicated, in one embodiment, the remote computing server 104 may also include one or more network and/or communications interface 207 for communicating with various computing entities, such as by communicating data, content, information, and/or similar terms used herein interchangeably that can be transmitted, received, operated on, processed, displayed, stored, and/or the like. Such communication may be executed using a wired data transmission protocol, such as fiber distributed data interface (FDDI), digital subscriber line (DSL), Ethernet, asynchronous transfer mode (ATM), frame relay, data over cable service interface specification (DOCSIS), or any other wired transmission protocol. Similarly, the remote computing server 104 may be configured to communicate via wireless external communication networks using any of a variety of protocols, such as general packet radio service (GPRS), Universal Mobile Telecommunications System (UMTS), Code Division Multiple Access 1900 (CDMA1900), CDMA1900 1X (1xRTT), Wideband Code Division Multiple Access (WCDMA), Global System for Mobile Communications (GSM), Enhanced Data rates for GSM Evolution (EDGE), Time Division-Synchronous Code Division Multiple Access (TD-SCDMA), Long Term Evolution (LTE), Evolved Universal Terrestrial Radio Access Network (E-UTRAN), Evolution-Data Optimized (EVDO), High Speed Packet Access (HSPA), High-Speed Downlink Packet Access (HSDPA), Institute of Electrical and Electronics Engineers (IEEE) 802.11 (Wi-Fi), Wi-Fi Direct, 802.16 (WiMAX), ultra-wideband (UWB), infrared (IR) protocols, near field communication (NFC) protocols, Wibree, Bluetooth protocols, wireless universal serial bus (USB) protocols, and/or any other wireless protocol. The remote computing server 104 may use such protocols and standards to communicate using Border Gateway Protocol (BGP), Dynamic Host Configuration Protocol (DHCP), Domain Name System (DNS), File Transfer Protocol (FTP), Hypertext Transfer Protocol (HTTP), HTTP over TLS/SSL/Secure, Internet Message Access Protocol (IMAP), Network Time Protocol (NTP), Simple Mail Transfer Protocol (SMTP), Telnet, Transport Layer Security (TLS), Secure Sockets Layer (SSL), Internet Protocol (IP), Transmission Control Protocol (TCP), User Datagram Protocol (UDP), Datagram Congestion Control Protocol (DCCP), Stream Control Transmission Protocol (SCTP), HyperText Markup Language (HTML), and/or the like.

As will be appreciated, one or more of the remote computing server's components may be located remotely from components of other remote computing servers, such as in a distributed system. Furthermore, one or more of the components may be aggregated and additional components performing functions described herein may be included in the remote computing server 104. Thus, the remote computing server 104 can be adapted to accommodate a variety of needs and circumstances.

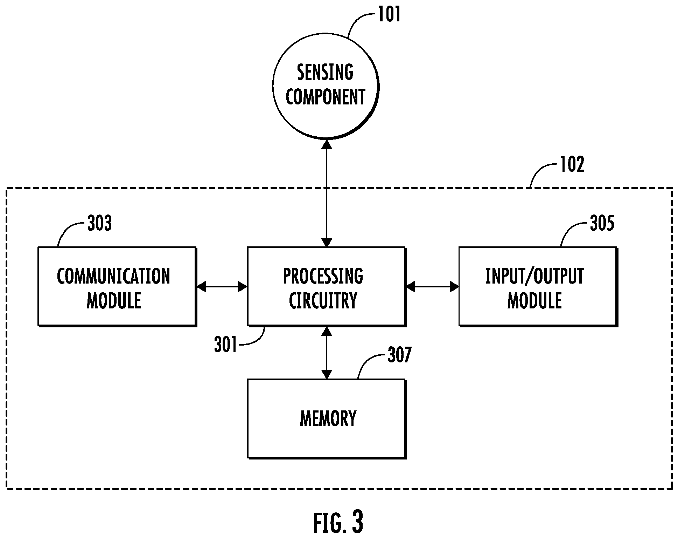

Referring now to FIG. 3, a schematic diagram depicting an example controller component 102 in accordance with various embodiments of the present disclosure. As shown, the controller component 102 comprises a processing circuitry 301, a communication module 303, an input/output module 305, a memory 307 and/or other components configured to perform various operations, procedures, functions or the like described herein.

As shown, the controller component 102 (such as the processing circuitry 301, communication module 303, input/output module 305 and memory 307) is electrically coupled to and/or in electronic communication with a sensing component 101. As depicted, the sensing component 101 may exchange (e.g., transmit and receive) data with the processing circuitry 301 of the controller component 102. For example, sensing component 101 may generate sensor data and transmit the sensor data to the processing circuitry 301.

The processing circuitry 301 may be implemented as, for example, various devices comprising one or a plurality of microprocessors with accompanying digital signal processors; one or a plurality of processors without accompanying digital signal processors; one or a plurality of coprocessors; one or a plurality of multi-core processors; one or a plurality of controllers; processing circuits; one or a plurality of computers; and various other processing elements (including integrated circuits, such as ASICs or FPGAs, or a certain combination thereof). In some embodiments, the processing circuitry 301 may comprise one or more processors. In one exemplary embodiment, the processing circuitry 301 is configured to execute instructions stored in the memory 307 or otherwise accessible by the processing circuitry 301. When executed by the processing circuitry 301, these instructions may enable the controller component 102 to execute one or a plurality of the functions as described herein. No matter whether it is configured by hardware, firmware/software methods, or a combination thereof, the processing circuitry 301 may comprise entities capable of executing operations according to the embodiments of the present invention when correspondingly configured. Therefore, for example, when the processing circuitry 301 is implemented as an ASIC, an FPGA, or the like, the processing circuitry 301 may comprise specially configured hardware for implementing one or a plurality of operations described herein. Alternatively, as another example, when the processing circuitry 301 is implemented as an actuator of instructions (such as those that may be stored in the memory 307), the instructions may specifically configure the processing circuitry 301 to execute one or a plurality of algorithms and operations described herein.

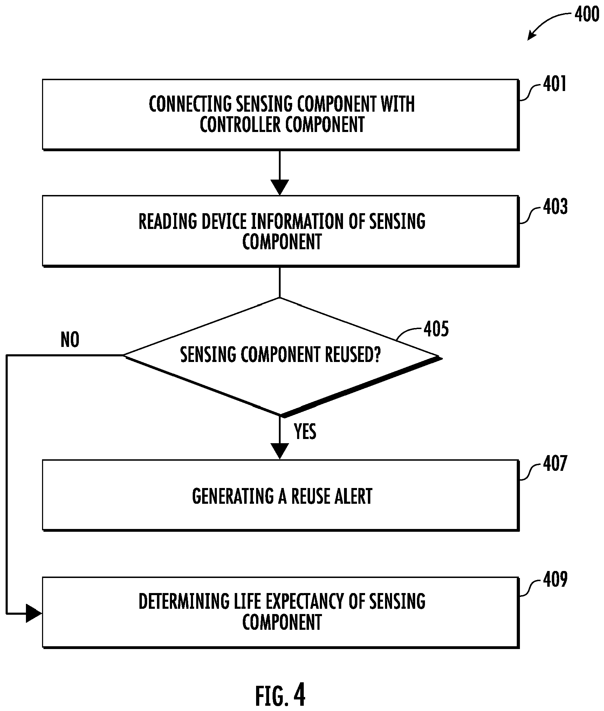

The memory 307 may comprise, for example, a volatile memory, a non-volatile memory, or a certain combination thereof. Although illustrated as a single memory in FIG. 4, the memory 307 may comprise a plurality of memory components. In various embodiments, the memory 307 may comprise, for example, a hard disk drive, a random-access memory, a cache memory, a flash memory, a Compact Disc Read-Only Memory (CD-ROM), a Digital Versatile Disk Read-Only Memory (DVD-ROM), an optical disk, a circuit configured to store information, or a certain combination thereof. The memory 307 may be configured to store information, data, application programs, instructions, and etc., so that the controller component 102 can execute various functions according to the embodiments of the present disclosure. For example, in at least some embodiments, the memory 307 is configured to cache input data for processing by the processing circuitry 301. Additionally, or alternatively, in at least some embodiments, the memory 307 is configured to store program instructions for execution by the processing circuitry 301. The memory 307 may store information in the form of static and/or dynamic information. When the functions are executed, the stored information may be stored and/or used by the controller component 102.

The communication module 303 may be implemented as any apparatus included in a circuit, hardware, a computer program product or a combination thereof, which is configured to receive and/or transmit data from/to another component or apparatus. The computer program product comprises computer-readable program instructions stored on a computer-readable medium (for example, the memory 307) and executed by a controller component 102 (for example, the processing circuitry 301). In some embodiments, the communication module 303 (as with other components discussed herein) may be at least partially implemented as the processing circuitry 301 or otherwise controlled by the processing circuitry 301. In this regard, the communication module 303 may communicate with the processing circuitry 301, for example, through a bus. The communication module 303 may comprise, for example, antennas, transmitters, receivers, transceivers, network interface cards and/or supporting hardware and/or firmware/software, and is used for establishing communication with another apparatus. The communication module 303 may be configured to receive and/or transmit any data that may be stored by the memory 307 by using any protocol that can be used for communication between apparatuses. The communication module 303 may additionally or alternatively communicate with the memory 307, the input/output module 305 and/or any other component of the controller component 102, for example, through a bus.

In some embodiments, the controller component 102 may comprise an input/output module 305. The input/output module 305 may communicate with the processing circuitry 301 to receive instructions input by the user and/or to provide audible, visual, mechanical or other outputs to the user. Therefore, the input/output module 305 may comprise supporting devices, such as a keyboard, a mouse, a display, a touch screen display, and/or other input/output mechanisms. Alternatively, at least some aspects of the input/output module 305 may be implemented on a device used by the user to communicate with the controller component 102. The input/output module 305 may communicate with the memory 307, the communication module 303 and/or any other component, for example, through a bus. One or a plurality of input/output modules and/or other components may be included in the controller component 102.

Referring now to FIG. 4, a flowchart diagram illustrating an example method 400 for detecting reuse of a sensing component, in accordance with various embodiments of the present disclosure is provided.

As depicted in FIG. 4, in some examples, the method 400 may be performed by a processing circuitry (for example, but not limited to, an application-specific integrated circuit (ASIC), a central processing unit (CPU)). In some examples, the processing circuitry may be electrically coupled to and/or in electronic communication with other circuitries of the example apparatus, such as, but not limited to, a sensing component 101, a memory (such as, for example, random access memory (RAM) for storing computer program instructions), and/or a display circuitry (for rendering information on a display).

In some examples, one or more of the procedures described in FIG. 4 may be embodied by computer program instructions, which may be stored by a memory (such as a non-transitory memory) of a system employing an embodiment of the present disclosure and executed by a processing circuitry (such as a processor) of the system. These computer program instructions may direct the system to function in a particular manner, such that the instructions stored in the memory circuitry produce an article of manufacture, the execution of which implements the function specified in the flow diagram step/operation(s). Further, the system may include one or more other circuitries. Various circuitries of the system may be electronically coupled between and/or among each other to transmit and/or receive energy, data and/or information.

In some examples, embodiments may take the form of a computer program product on a non-transitory computer-readable storage medium storing computer-readable program instruction (e.g., computer software). Any suitable computer-readable storage medium may be utilized, including non-transitory hard disks, CD-ROMs, flash memory, optical storage devices, or magnetic storage devices.

At step/operation 401 of the example method 400, a processing circuitry (such as, but not limited to, the processing circuitry 301 of the controller component 102 illustrated in connection with FIG. 3, discussed above) connects the sensing component 101 with the controller component 102. For example, the sensing component 101 is removable from and attachable to the controller component 102. In some examples, the sensing component 101 is configured to be in an electrical communication with the controller component 102 when attached to the controller component 102.

In some examples, the sensing component 101 may be a flow sensing component, which is configured to measure a flow rate of a flowing media.

In some examples, the processing circuitry 301 of the controller component 102 may be configured to notify users in an instance in which the sensing component 101 is successfully connected to the controller component 102.

At step/operation 403 of the example method 400, the processing circuitry (such as, but not limited to, the processing circuitry 301 of the controller component 102 illustrated in connection with FIG. 3, discussed above) reads device information of the sensing component 101. For example, the processing circuitry 301 of the controller component 102 may read device information of the sensing component 101, such as an identification (ID) of the sensing component 101 and calibration parameters of the sensing component 101.

In some examples, the calibration parameters of the sensing component 101 may include a residual life the sensing component 101. In some examples, the calibration parameters of the sensing component may further include an ambient temperature, an average operating flow, and a transient flow of the sensing component 101.

At step/operation 405 of the example method 400, the processing circuitry (such as, but not limited to, the processing circuitry 301 of the controller component 102 illustrated in connection with FIG. 3, discussed above) determines if the sensing component 101 is reused based on the device information.

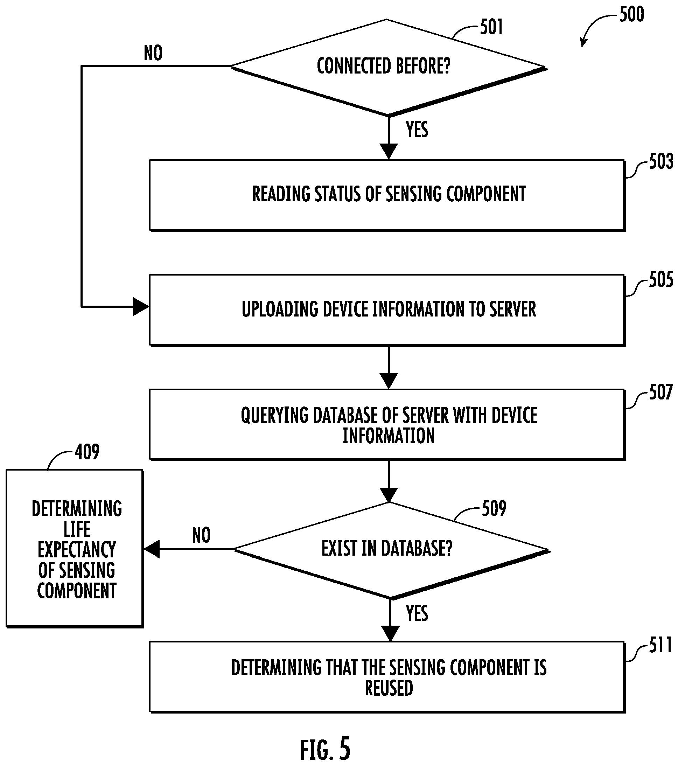

Referring now to FIG. 5, a flowchart diagram illustrating an example method 500 of performing the step/operation 405 to determine if the sensing component 101 is reused based on the device information is provided.

At step/operation 501 of the example method 500, a processing circuitry (such as, but not limited to, the processing circuitry 301 of the controller component 102 illustrated in connection with FIG. 3, discussed above) determines if the sensing component 101 has previously been connected to a remote computing server 104. If the processing circuitry 301 of the controller component 102 determines that the sensing component 101 has been previously connected to the remote computing server 104, the example method may proceed to step/operation 503. If the processing circuitry 301 of the controller component 102 determines that the sensing component 101 has not been previously connected to the remote computing server 104, the example method may proceed to step/operation 505.

At step/operation 503 of the example method 500, the processing circuitry (such as, but not limited to, the processing circuitry 301 of the controller component 102 illustrated in connection with FIG. 3, discussed above) uploads the device information to the remote computing server 104.

At step/operation 505 of the example method 500, the processing circuitry (such as, but not limited to, the processing circuitry 301 of the controller component 102 illustrated in connection with FIG. 3, discussed above) reads a status of the sensing component 101 from the remote computing server 104.

In some embodiments, the status of the sensing component 101 may include device information of the sensing component 101, calibration parameters of the sensing component 101, and a life expectancy of the sensing component from a previous detection. For example, a life expectancy of the sensing component 101 may be provided at an expected average flow rate, an ambient temperature, and/or an ambient humidity.

At step/operation 507 of the example method 500, the processing circuitry (such as, but not limited to, the processing circuitry 301 of the controller component 102 illustrated in connection with FIG. 3, discussed above) queries a database of the remote computing server 104 with the device information.

At step/operation 509 of the example method 500, a processing circuitry (such as, but not limited to, the processing circuitry 301 of the controller component 102 illustrated in connection with FIG. 3, discussed above) determines if the device information exists in the database of the remote computing server 104. If the processing circuitry 301 of the controller component 102 determines that the device information exists in the database of the remote computing server 104, the example method may proceed to step/operation 511. If the processing circuitry 301 of the controller component 102 determines that the device information does not exist in the database of the server, the example method may proceed to step/operation 409.

For example, if the processing circuitry 301 of the controller component 102 determines that the device information of the sensing component 101 exists in the database of remote computing server 104, the processing circuitry 301 of the controller component 102 may determine the sensing component 101 has been previously connected with the remote computing server 104, and the example method may proceed to step/operation 511.

For example, if the processing circuitry 301 of the controller component 102 determines that the device information of the sensing component 101 does not exist in the database of remote computing server 104, the processing circuitry 301 of the controller component 102 may determine the sensing component 101 has not been previously connected with the remote computing server 104, and the example method may proceed to step/operation 409.

At step/operation 511 of the example method 500, the processing circuitry (such as, but not limited to, the processing circuitry 301 of the controller component 102 illustrated in connection with FIG. 3, discussed above) determines that the sensing component is reused.

In various embodiments, if the processing circuitry 301 of the controller component 102 determines that the sensing component 101 is reused based on the device information, the example method may proceed to step/operation 407. If the processing circuitry 301 of the controller component 102 determines that the sensing component 101 is not reused based on the device information, the example method may proceed to step/operation 409.

Referring back to FIG. 4, at step/operation 407 of the example method 400, the processing circuitry (such as, but not limited to, the processing circuitry 301 of the controller component 102 illustrated in connection with FIG. 3, discussed above) generates a reuse alert to users.

In some examples, the controller component 102 may be configured to generate the reuse alert to users in real-time in an instance in which the sensing component 101 is reused. In some examples, the reuse alert may be a visual indicator and/or an acoustic indicator. Additionally, in some examples, the controller component 102 may be configured to send an instruction for replacing the sensing component 101 along with the reuse alert to users in an instance in which the sensing component 101 is reused.

At step/operation 409 of the example method 400, the processing circuitry (such as, but not limited to, the processing circuitry 301 of the controller component 102 illustrated in connection with FIG. 3, discussed above) determines a life expectancy of the sensing component 101.

In various embodiments, the processing circuitry 301 of the controller component 102 may depends on a sever 104 to perform the steps/operations 405, 407, and 409.

In some examples, the processing circuitry 301 of the controller component 102 may be configured to continuously monitor a connection status between the controller component 102 and the sensing component 101. For example, the steps/operations of the example method 400 may be triggered to be performed by an event, such as a connecting between the controller component 102 and the sensing component 101.

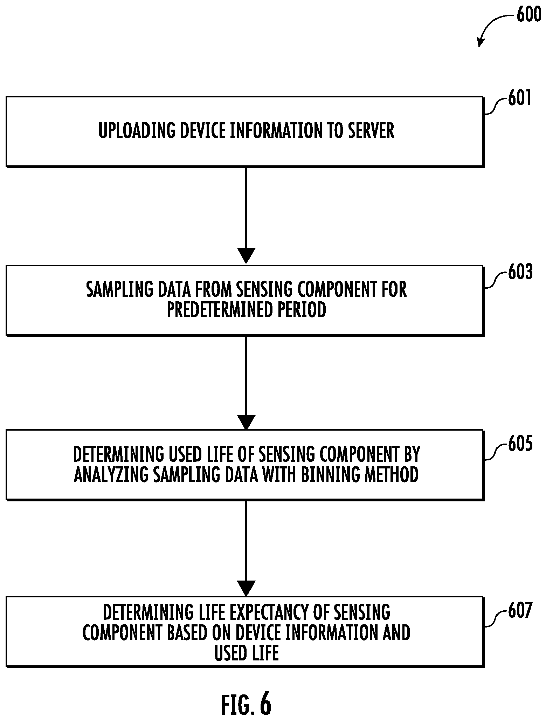

Referring now to FIG. 6, a flowchart diagram illustrating an example method 600 of performing the step/operation 409 to determine a life expectancy of the sensing component 101 is provided.

At step/operation 601 of the example method 600, a processing circuitry (such as, but not limited to, the processing circuitry 301 of the controller component 102 illustrated in connection with FIG. 3, discussed above) uploads the device information of the sensing component 101 to the remote computing server 104.

At step/operation 603 of the example method 600, the processing circuitry (such as, but not limited to, the processing circuitry 301 of the controller component 102 illustrated in connection with FIG. 3, discussed above) collects data from the sensing component 101 for a predetermined period.

In some embodiments, the predetermined period may be in a range of 1-60 minutes. In some examples, the predetermined period may be 10 minutes. For example, the processing circuitry 301 of the controller component 102 may collect data from the sensing component 101 on a 10-minute interrupt.

At step/operation 605 of the example method 600, the processing circuitry (such as, but not limited to, the processing circuitry 301 of the controller component 102 illustrated in connection with FIG. 3, discussed above) determines a used life of the sensing component 101 by analyzing the collected data with a binning method.

In various embodiments, the binning method is a way to group a number of more or less continuous values into a smaller number of bins. For example, the collected data may include a continuous flow rate in a range, and the arrange may be divided into a number of flow rate intervals, which is called bins. The collected data may be analyzed by counting a frequency of the flow rate in each of the bins.

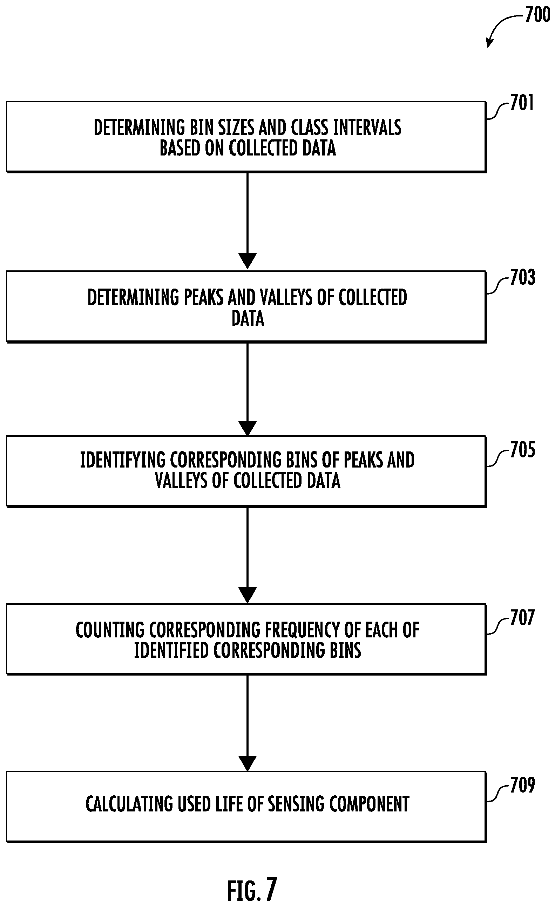

Referring now to FIG. 7, a flowchart diagram illustrating an example method 700 of performing the step/operation 605 to determine the used life of the sensing component 101 by analyzing the collected data with a binning method is provided.

At step/operation 701 of the example method 700, a processing circuitry (such as, but not limited to, the processing circuitry 301 of the controller component 102 illustrated in connection with FIG. 3, discussed above) determines bin sizes and class intervals based on the collected data.

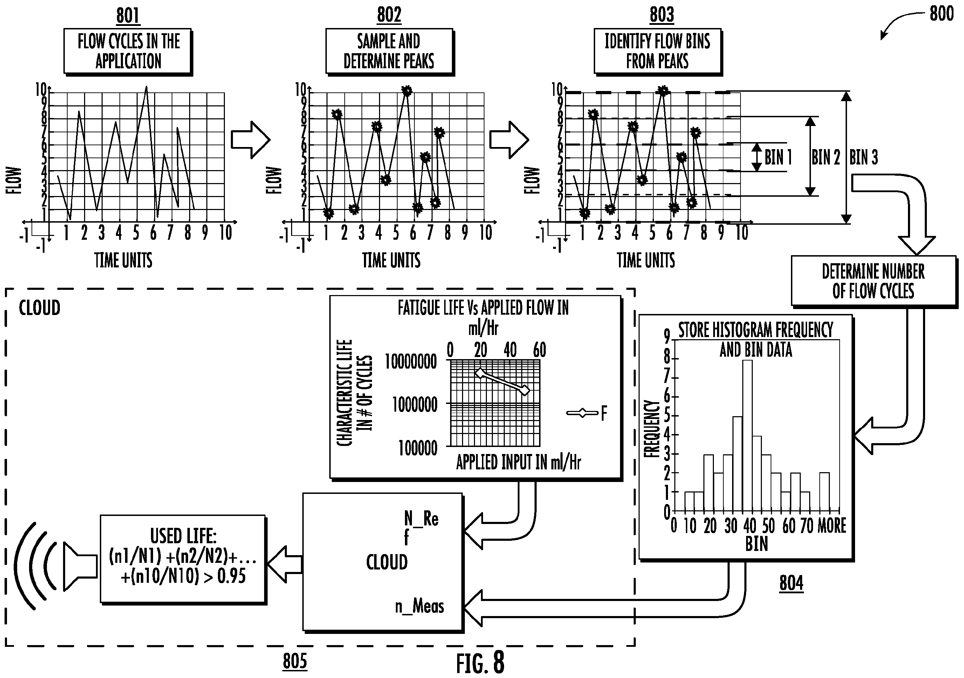

In various embodiments, as shown in 801 of FIG. 8, an example measured flow is illustrated based on the collected data from the sensing component 101. In some examples, the example measured flow varies with the time.

For example, the example measured flow may be in a range from 0 to 10. In some examples, the example measured flow may be categorized into three bins. For example, the first bin Bin1 may have a maximum flow range of 4-6. For example, the second bin Bin2 may have a maximum flow range of 2-8. For example, the third bin Bin3 may have a maximum flow range of 0-10.

At step/operation 703 of the example method 700, the processing circuitry (such as, but not limited to, the processing circuitry 301 of the controller component 102 illustrated in connection with FIG. 3, discussed above) determines peaks and valleys of the collected data.

In various embodiments, as shown in 802 of FIG. 8, the peaks and valleys of the example measured flow is determined and circled out.

At step/operation 705 of the example method 700, the processing circuitry (such as, but not limited to, the processing circuitry 301 of the controller component 102 illustrated in connection with FIG. 3, discussed above) identifies corresponding bins of the peaks and the valleys of the collected data.

In various embodiments, as shown in 803 of FIG. 8, corresponding bins of the peaks and the valleys are determined based on the flow value of the peaks and valleys. For example, a peak of the flow value at 5 has a corresponding bin with a range of 4-6.

At step/operation 707 of the example method 700, the processing circuitry (such as, but not limited to, the processing circuitry 301 of the controller component 102 illustrated in connection with FIG. 3, discussed above) counts a corresponding frequency of each of the identified corresponding bins.

In various embodiments, a corresponding frequency of an identified bin may be determined by counting how many times the peaks and/or valleys fall within the corresponding range of the identified bin.

In various embodiments, as shown in 804 of FIG. 8, an example histogram frequency of bins is illustrated.

At step/operation 709 of the example method 700, the processing circuitry (such as, but not limited to, the processing circuitry 301 of the controller component 102 illustrated in connection with FIG. 3, discussed above) calculates the used life of the sensing component 101 based on the identified corresponding bins and the corresponding frequency of each of the identified corresponding bins.

In various embodiments, the processing circuitry 301 of the controller component 102 may be further configured to store and/or update the used life of the sensing component 101. In various embodiments, the processing circuitry 301 of the controller component 102 may be further configured to store and/or update the used life of the sensing component 101 on the remote computing server 104.

In various embodiments, as shown in 805 of FIG. 8, the used life of the sensing component 101 may be calculated based on the Eq. 1.

For example, a pattern of use of the sensing component 101 may be divided into three bins, where the first bin Bin1 corresponds to 0.2 cycle, the second bin Bin2 corresponds to 0.6 cycle, and the third bin Bin3 corresponds to 1 cycle. For example, the frequency of the first bin Bin1 may be 2, the frequency of the second bin Bin2 may be 2, and the frequency of the third bin Bin3 may be 4. The used life of the sensing component 101 may be calculated based on the Eq. 1:

$$\text{used life} = 0.2 * 2 + 0.6 * 2 + 1 * 4 = 5.6$$

In some examples, the used life of the sensing component 101 may be a number of cycles that the sensing component 101 has been operated. In some examples, the used life of the sensing component 101 may be a number of hours that the sensing component 101 has been operated.

Referring back to FIG. 6, at step/operation 607 of the example method 600, the processing circuitry (such as, but not limited to, the processing circuitry 301 of the controller component 102 illustrated in connection with FIG. 3, discussed above) determine the life expectancy of the sensing component 101 based on the device information and the used life.

In various embodiments, the processing circuitry 301 of the controller component 102 may be further configured to store and/or update the life expectancy of the sensing component 101. In various embodiments, the processing circuitry 301 of the controller component 102 may be further configured to store and/or update the life expectancy of the sensing component 101 on the remote computing server 104.

In various embodiments, the processing circuitry 301 of the controller component 102 may be further configured to send out an alert when the life expectancy is smaller than a predetermined threshold. In some examples, the alert may be a visual indicator and/or an acoustic indicator. In some examples, the alert may warn the users that the sensing component 101 is expiring and needs to be replaced.

In various embodiments, the processing circuitry 301 of the controller component 102 may be configured to display an indicator to indicate the used life of the sensing component 101 to users. In some examples, the users may set diagnostics actions according to the used life of the sensing component 101.

In various embodiments, the processing circuitry 301 of the controller component 102 may be further configured to display an indicator to indicate the life expectancy of the sensing component 101 to users. In some examples, the users may set diagnostics actions according to the life expectancy of the sensing component 101.

In various embodiments, the processing circuitry 301 of the controller component 102 may depends on the remote computing server 104 to perform the steps/operations 605 and 607.

Many modifications and other embodiments of the present disclosure set forth herein will come to mind to one skilled in the art to which these embodiments pertain having the benefit of the teachings presented in the foregoing descriptions and the associated drawings. Therefore, it is to be understood that the disclosure is not to be limited to the specific embodiments disclosed and that modifications and other embodiments are intended to be included within the scope of the appended claims. Moreover, although the foregoing descriptions and the associated drawings describe example embodiments in the context of certain example combinations of elements and/or functions, it should be appreciated that different combinations of elements and/or functions may be provided by alternative embodiments without departing from the scope of the appended claims. In this regard, for example, different combinations of elements and/or functions than those explicitly described above are also contemplated as may be set forth in some of the appended claims. Although specific terms are employed herein, they are used in a generic and descriptive sense only and not for purposes of limitation.

**Claims**

1. A method for detecting reuse of a sensing component by a controller component, comprising:

   reading device information of the sensing component;
   determining if the sensing component is reused based on the device information;
   in an instance in which the sensing component is reused, generating a reuse alert; and
   in an instance in which the sensing component is not reused, determining a life expectancy of the sensing component.

2. The method according to claim 1, wherein determining if the sensing component is reused based on the device information comprises:

   determining if the sensing component is connected to a server before;
   in an instance in which the sensing component has not been connected to the server before:

      uploading the device information to the server;
      querying a database of the server with the device information;
      determining if the device information exists in the database of the serve; and
      in an instance in which the device information exists in the database of the server, determining that the sensing component is reused.

3. The method according to claim 1, wherein determining the life expectancy of the sensing component comprises:

   uploading the device information to a server;
   collecting data from the sensing component for a predetermined period;
   determining a used life of the sensing component by analyzing the collected data with a binning method; and
   determining the life expectancy of the sensing component based on the device information and the used life.

4. The method according to claim 3, wherein the device information includes an identification of the sensing component and parameters of the sensing component.

5. The method according to claim 4, wherein the parameters of the sensing component includes an ambient temperature, an operating flow, and a transient flow of the sensing component.

6. The method according to claim 3, wherein analyzing the collected data with the binning method composes:

   determining bin sizes and class intervals based on the collected data;
   determining peaks and valleys of the collected data;
   identifying corresponding bins of the peaks and the valleys of the collected data;
   counting a corresponding frequency of each of the identified corresponding bins; and
   calculating the used life of the sensing component based on the identified corresponding bins and the corresponding frequency of each of the identified corresponding bins.

7. The method according to claim 3, wherein the sensing component is a flow sensing component configured to measure a flow rate of a flowing media.

8. The method according to claim 1, further comprising connecting the sensing component with the controller component, wherein:

   the sensing component is attachable to the controller component, and
   the sensing component is configured to be in an electrical communication with the controller component when attached.

9. The method according to claim 1, wherein the controller component includes a signal conditioner.

10. The method according to claim 9, wherein the signal conditioner is an amplifier or an analog-to-digital converter (ADC).

11. The method according to claim 1, further comprising:
storing the life expectancy of the sensing component.

12. The method according to claim 1, further comprising:
updating the life expectancy of the sensing component on a server.

13. The method according to claim 1, wherein the controller component is further configured to send the reuse alert to a user in rea-time.

14. A sensor, comprising:

a sensing component, and
a controller component, wherein the sensing component is attachable to the controller component, and the controller component is configured to:

read device information of the sensing component;
determine if the sensing component is reused based on the device information;
in an instance in which the sensing component is reused, generate a reuse alert; and
in an instance in which the sensing component is not reused, determine a life expectancy of the sensing component.

15. The sensor according to claim 14, wherein to determine if the sensing component is reused based on the device information, the controller component is configured to:

determine if the sensing component is connected to a server before;
in an instance in which the sensing component has not been connected to the server before;
upload the device information to the server;
query a database of the server with the device information;
determine if the device information exists in the database of the serve; and
in an instance in which the device information exists in the database of the server, determine that the sensing component is reused.

FIG. 1

104

PROCESSOR
COMPONENT — 201

205

STORAGE MEDIA

203

MEMORY ELEMENT

COMMUNICATIONS
INTERFACE — 207

FIG. 2

FIG. 3

400

```
┌─────────────────────────────────────┐
│  CONNECTING SENSING COMPONENT WITH   │ ── 401
│        CONTROLLER COMPONENT          │
└─────────────────────────────────────┘
                  │
                  ▼
┌─────────────────────────────────────┐
│  READING DEVICE INFORMATION OF SENSING│ ── 403
│              COMPONENT               │
└─────────────────────────────────────┘
                  │
                  ▼
        NO      ╱◇╲                    ── 405
  ┌──────────  SENSING COMPONENT REUSED?  ╲
  │             ╲        ╱
  │               ╲    ╱
  │                 YES
  │                  ▼
  │      ┌─────────────────────────────┐
  │      │     GENERATING A REUSE ALERT │ ── 407
  │      └─────────────────────────────┘
  │
  │      ┌─────────────────────────────┐
  └─────▶│ DETERMINING LIFE EXPECTANCY OF SENSING │ ── 409
         │          COMPONENT           │
         └─────────────────────────────┘
```

FIG. 4

FIG. 5

600

| UPLOADING DEVICE INFORMATION TO SERVER | 601 |

| SAMPLING DATA FROM SENSING COMPONENT FOR PREDETERMINED PERIOD | 603 |

| DETERMINING USED LIFE OF SENSING COMPONENT BY ANALYZING SAMPLING DATA WITH BINNING METHOD | 605 |

| DETERMINING LIFE EXPECTANCY OF SENSING COMPONENT BASED ON DEVICE INFORMATION AND USED LIFE | 607 |

FIG. 6

700

701
DETERMINING BIN SIZES AND CLASS INTERVALS
BASED ON COLLECTED DATA

703
DETERMINING PEAKS AND VALLEYS OF COLLECTED
DATA

705
IDENTIFYING CORRESPONDING BINS OF PEAKS AND
VALLEYS OF COLLECTED DATA

707
COUNTING CORRESPONDING FREQUENCY OF EACH OF
IDENTIFIED CORRESPONDING BINS

709
CALCULATING USED LIFE OF SENSING COMPONENT

FIG. 7

**801** FLOW CYCLES IN THE APPLICATION

**802** SAMPLE AND DETERMINE PEAKS

**803** IDENTIFY FLOW BINS FROM PEAKS

800

DETERMINE NUMBER OF FLOW CYCLES

CLOUD

FATIGUE LIFE Vs APPLIED FLOW IN ml/Hr

STORE HISTOGRAM FREQUENCY AND BIN DATA

**804**

USED LIFE: $(n1/N1) + (n2/N2) + ... + (n10/N10) > 0.95$

$N\_Re$
$f$
CLOUD
$n\_Meas$

**805**

FIG. 8

EP 4 432 298 A1

Europäisches
Patentamt

European
Patent Office

Office européen
des brevets

# EUROPEAN SEARCH REPORT

Application Number

EP 24 15 8485

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2022/334942 A1 (EGOROVA MARTA [US] ET AL) 20 October 2022 (2022-10-20)<br>* paragraphs [0013], [0039] - paragraph [0082] *<br>* figures 1-6 * | 1-15 | INV.<br>G16H40/60<br>G05B21/00<br>G05B23/00 |
| A | WO 2021/231326 A1 (MASIMO CORP [US]) 18 November 2021 (2021-11-18)<br>* paragraph [0048] - paragraph [0260] *<br>* figures 1-2c,11a-17 * | 1-15 | |

TECHNICAL FIELDS
SEARCHED      (IPC)

G05B
G06Q
G16H

The present search report has been drawn up for all claims

| Place of search | Date of completion of the search | Examiner |
|---|---|---|
| Munich | 18 July 2024 | Schechner-Resom, G |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons
................................................................
& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 24 15 8485

This annex lists the patent family members relating to the patent documents cited in the above-mentioned European search report.
The members are as contained in the European Patent Office EDP file on
The European Patent Office is in no way liable for these particulars which are merely given for the purpose of information.

18-07-2024

| Patent document cited in search report | | Publication date | Patent family member(s) | | Publication date |
|---|---|---|---|---|---|
| US 2022334942 | A1 | 20-10-2022 | CN 115222347 A | | 21-10-2022 |
| | | | US 2022334942 A1 | | 20-10-2022 |
| WO 2021231326 | A1 | 18-11-2021 | CN 115916033 A | | 04-04-2023 |
| | | | EP 4149347 A1 | | 22-03-2023 |
| | | | JP 2023525563 A | | 16-06-2023 |
| | | | WO 2021231326 A1 | | 18-11-2021 |

EPO FORM P0459

For more details about this annex : see Official Journal of the European Patent Office, No. 12/82